# EUROPEAN PATENT SPECIFICATION

(11) **EP 2 087 137 B1**
(45) Date of publication and mention of the grant of the patent: **30.11.2011**
(21) Application number: 07825755.7
(22) Date of filing: 15.10.2007
(51) Int. Cl.: C12Q 1/68

(54) **A MOLECULE FOR PROVIDING A STANDARD FOR THE QUANTITATIVE ANALYSIS OF THE METHYLATION STATUS OF A NUCLEIC ACID**
MOLEKÜL ZUR BEREITSTELLUNG EINES STANDARDS ZUR QUANTITATIVEN ANALYSE DES METHYLIERUNGSSTATUS EINER NUKLEINSÄURE
MOLÉCULE POUR ÉLABORER UN ÉTALON D'ANALYSE QUANTITATIVE DE L'ÉTAT DE MÉTHYLATION D'UN ACIDE NUCLÉIQUE

(30) Priority: 18.10.2006 EP 06122484; 07.11.2006 EP 06123628
(43) Date of publication of application: 12.08.2009
(73) Proprietor: Epigenomics AG, 10178 Berlin (DE)
(72) Inventor: SCHATZ, Philipp, 10435 Berlin (DE)
(74) Representative: Zwicker, Jörk
(86) International application number: PCT/IB2007/003673
(87) International publication number: WO 2008/047234

(56) References cited:
- WO-A-2004/013284
- WO-A-2005/040399
- WO-A-2005/075671
- WO-A-2005/090597
- WO-A-2005/098035

## Description

The invention relates to a nucleic acid for providing a standard for the quantitative bisulfite based analysis of the methylation status of a DNA molecule according to claim 1, to the use of such a nucleic acid according to claim 10, to a method according to claim 11, and to a kit for the realization of the method according to claim 14.

Throughout this application, various publications are cited. The disclosure of these publications is hereby incorporated by reference in its entirety into this application to describe more fully the state of the art to which this invention pertains.

Gene regulation has been correlated with methylation of a gene or genome. Certain cell types consistently display specific methylation patterns, and this has been shown for a number of different cell types (Adorjan et al. (2002) Tumor class prediction and discovery by microarray-based DNA methylation analysis. Nucleic Acids Res 30(5) e21).

In higher order eukaryotes, DNA is methylated nearly exclusively at cytosine bases located 5' to guanine in the CpG dinucleotide. This modification has important regulatory effects on gene expression, especially when involving CpG rich areas, known as CpG islands, located in the promoter regions of many genes. While almost all gene-associated islands are protected from methylation on autosomal chromosomes, extensive methylation of CpG islands has been associated with transcriptional inactivation of selected imprinted genes and genes on the inactive X-chromosome of females.

The modification of cytosine in the form of methylation contains significant information. The identification of 5-methylcytosine within a DNA sequence is of importance in order to uncover its role in gene regulation. The position of a 5-methylcytosine cannot be identified by a normal sequencing reaction, since it behaves just as an unmethylated cytosine as per its hybridization preference.

In any standard amplification, such as a standard polymerase chain reaction (PCR), this relevant epigenetic information will be lost.

Several methods are known to solve this problem. Generally, genomic DNA is treated with a chemical or enzyme leading to a conversion of the cytosine bases, which consequently allows to distinguish between methylated and unmethylated cytosine bases. The most common methods are a) the use of methylation-sensitive restriction enzymes capable of differentiating between methylated and unmethylated DNA and b) the treatment with bisulfite. The use of methylation-sensitive restriction enzymes is limited due to the selectivity of the restriction enzyme towards a specific recognition sequence.

Therefore, bisulfite treatment, allowing for the specific reaction of bisulfite with cytosine, which upon subsequent alkaline hydrolysis is converted to uracil, whereas 5-methylcytosine remains unmodified under these conditions (Shapiro et al. (1970) Nature 227: 1047) is currently the most frequently used method for analyzing DNA for the presence of 5-methylcytosine. Uracil corresponds to thymine in its base pairing behavior, that is, it hybridizes to adenine. 5-methylcytosine does not change its chemical properties under this treatment and therefore still hybridizes with guanine. Consequently, the treated DNA is converted in such a manner that 5-methylcytosine, which originally could not be distinguished from cytosine by its hybridization behavior, can now be detected as the only remaining cytosine using conventional molecular biological techniques, such as amplification and hybridization or sequencing.

An overview of the further known methods of detecting 5-methylcytosine may be gathered from the following review article: Fraga FM and Esteller M, Biotechniques (2002) 33(3): 632, 634, 636-49.

As the use of methylation-specific enzymes is dependent on the presence of restriction sites, most methods are based on a bisulfite treatment that is conducted before a detection or amplifying step (for review: DE 100 29 915 A1, page 2, lines 35-46 or the according translated US application 10/311,661; see also WO 2004/067545). The term 'bisulfite treatment' is meant to comprise treatment with a bisulfite, a disulfite or a hydrogensulfite solution. As known to the expert skilled in the art and according to the invention, the term "bisulfite" is used interchangeably for "hydrogensulfite".

Several laboratory protocols are known in the art, all of which comprise of the following steps: The genomic DNA is isolated, denatured, converted several hours by a concentrated bisulfite solution and finally desulfonated and desalted (e.g.: Frommer et al. (1992) A genomic sequencing protocol that yields a positive display of 5-methylcytosine residues in individual DNA strands. Proc Natl Acad Sci USA 89(5): 1827-1831).

Subsequent to a bisulfite treatment, usually short, specific fragments of a known gene are amplified and either completely sequenced (Olek A, Walter J. (1997) The pre-implantation ontogeny of the H19 methylation imprint. Nat Genet 3: 275-276) or individual cytosine positions are detected by a primer extension reaction (Gonzalgo ML and Jones PA (1997) Rapid quantitation of methylation differences at specific sites using methylation-sensitive single nucleotide primer extension (Ms-SNuPE Nucleic Acids Res 25: 2529-2531, WO 95/00669) or by enzymatic digestion (Xiong Z, Laird PW. (1997) COBRA: a sensitive and quantitative DNA methylation assay. Nucleic Acids Res 25: 2535-2534).

Another technique to detect hypermethylation is the so-called methylation specific PCR (MSP) (Herman JG, Graff JR, Myohanen S, Nelkin BD and Baylin SB. (1996), Methylation-specific PCR: a novel PCR assay for methylation status of CpG islands. Proc Natl Acad Sci USA 93: 9821-9826). The technique is based on the use of primers that differentiate between a methylated and a non-methylated sequence if applied after bisulfite treatment of said DNA sequence. The primer either contains a guanine at the position corresponding to the cytosine in which case it will after bisulfite treatment only bind if the position was methylated. Or the primer contains an adenine at the corresponding cytosine position and therefore only binds to said DNA sequence after bisulfite treatment if the cytosine was unmethylated and has hence been altered by the bisulfite treatment so that it hybridizes to adenine. With the use of these primers, amplicons can be produced specifically depending on the methylation status of a certain cytosine and will as such indicate its methylation state.

Another technique is the detection of methylation via a labeled probe, such as used in the so called Taqman^{®} PCR, also known as MethyLight (US 6,331,393 B1). With this technique it is feasible to determine the methylation state of single or of several positions directly during PCR, without having to analyze the PCR products in an additional step.

Furthermore, detection by hybridization has also been described (Olek et al., WO 99/28498).

The treatment with bisulfite (or similar chemical agents or enzymes) with the effect of altering the base pairing behavior of one type of cytosine specifically, either the methylated or the unmethylated, thereby introducing different hybridization properties, makes the treated DNA more applicable to the conventional methods of molecular biology, especially the polymerase-based amplification methods, such as PCR.

In order to use any of the methods mentioned above to analyze the methylation status of a nucleic acid in a quantitative fashion, a reference or standard is needed for the amount of methylated versus unmethylated nucleic acid.

Thus far, a common approach for providing such a reference was to perform a whole genome amplification (MDA) of genomic DNA. The amplified DNA is then treated with SssI methyltransferase to generate a DNA that is methylated at every cytosine position (100 % methylated). In parallel, a mock treatment is performed without SssI methyltransferase, which yields a DNA that is completely unmethylated (0 % methylated). The standard is then prepared by mixing methylated and unmethylated DNA in defined ratios, such as 0 %, 25 %, 50 %, 75%, 100% methylated etc.

Different standards for the quantitative analysis of the methylation status of nucleic acid have been disclosed in WO 2005/090597, WO 2005/098035, WO 2005/075671, WO 2004/013284, and WO 2005/040399.

WO 2005/090597 discloses a synthetic standard for the quantitative analysis of the methylation status of a nucleic acid. The standard is prepared by mixing at least two DNA samples, one of them being completely unmethylated and the other one being completely methylated. The level of proportion can be shifted by the mixing ratio of the sample, each mixing ratio resulting in another calibrations standard. The standard disclosed in WO 2005/098035 corresponds to DNA's with different degrees of methylation. The different degrees of methylation are obtained by mixing together in different ratios methylated and unmethylated DNA. In WO 2005/075671 the standard is prepared by whole gene amplification with random primers and in the presence of unmethylated cytosintriphosphate. After several rounds of amplification only unmethylated DNA is obtained that is used as standard.

WO 2004/013284 discloses an internal standard for the quantitative analysis of the methylation status of a nucleic acid. The standard corresponds to ¹⁵N₃2'-deoxycytidine that is added as the internal standard for 2'-deoxycytidine and to methyl-D3, ring-6-D1 5-methyl-2'deoxycytidine for 5-methyl-2'-deoxycytidine. Finally, the standard disclosed in WO 2005/040399 corresponds to a mixture of DNAs corresponding to different degree of methylation obtained by treating DNA with an active methylase for shorter periods of time than is necessary to achieve complete methylation.

The preparation of bisulfite DNA standards is critical because methods with which one could ensure the quality of the source DNA are lacking. It is, for example not possible to assess easily whether the MDA reaction was successful and with which efficiency the SssI methyltransferase treatment was performed. Therefore, the failure rate when mixtures of methylated and unmethylated DNA are prepared are high, which yields quantification problematic.

Furthermore, no suitable method is known that could be used to ensure that the overall methylation level of the mixtures of 100 % methylated and 0 % methylated DNA, e.g. 50 % overall methylation, is correct.

It is also not possible to produce a standard generated by mixing methylated with unmethylated DNA at certain ratios in large batches. Instead, the standard needs to be produced again and again. In addition, mixing of two components introduces a further error.

According to that, he problem underlying the present invention therefore was to provide a standard that could be used with known methods for quantitatively analyzing the methylation status of a given DNA molecule in an easy and reliable fashion.

Surprisingly, the inventor was able to solve this problem by inventing the present nucleic acid and the present method. The central idea of the invention is to provide a single molecule that generates signals for both the methylated and the unmethylated status in a fixed ratio. This can be achieved by providing molecule with a cassette-like structure. Each cassette contains at least one means for providing a signal for either the methylated or the unmethylated state of a cytosine base. The standard molecule can contain a different number of cassettes in a particular ratio, whereby any particular signal ratio of methylated over unmethylated cytosine bases can be provided. Quality control of the standard nucleic acid can easily be performed using sequencing. Accordingly, the inventor was also able to provide a method that makes use of this standard molecule.

### Description of the invention

The underlying problem is solved by a nucleic acid that is provided as a standard for the quantitative bisulfite based analysis of the methylation status of a DNA molecule, in particular of a genomic DNA molecule, with a given sequence. This given sequence contains at least one CpG position that is to be analyzed for its methylation status using said nucleic acid.

For this purpose, the nucleic acid comprises a first sequence portion and a second sequence portion, both of which comprise at least part of the given sequence with at least one CpG position, the CpG position of interest. In the first sequence portion, all of the CpG positions represent the methylated status, and all of those CpG positions will therefore generate at least one detectable control signal of the methylated status. In the second sequence portion of the nucleic acid, all of the CpG positions represent the unmethylated status, and therefore, all of those CpG positions will generate at least one detectable control signal of the unmethylated status.

In other words, the nucleic acid used as a standard comprises at least two sequence portions in one molecule, each generating a detectable signal representing a particular methylation status, whereby at least one of the sequence portions represents the methylated, and at least one other sequence portion represents the unmethylated

Preferably, the nucleic acid used as a standard comprises the sequence of interest (containing at least one CpG position that is to be analyzed) at least twice, namely one time with a sequence of said at least one CpG position that represents the methylated status, and one time with a sequence of said at least one CpG position that represents the unmethylated status. In other words, the first and the second sequence portion of the nucleic acid comprise the same part of the given sequence of the DNA molecule to be analyzed, i.e. the sequences of the first and second sequence portions are identical save for the sequences at the CpG positions.

In a preferred embodiment of the nucleic acid according to the invention, the nucleic acid comprises more than one first sequence portion and/or more than one second sequence portion. The first sequence portion and the second sequence portion can be contained in the nucleic acid in a fixed ratio, such as 1:1, 2:1, 3:1, 1:2, or 1:3 (ratio of the first sequence portion to the second sequence portion). This way, the nucleic acid can generate signals, e.g., for 0 %, 25 %, 50 %, 75 %, or 100 % methylation.

When bisulfite is used to treat the DNA molecule that is to be analyzed for its methylation state, the methylated CpG positions of the nucleic acid are represented by a CG. In contrast, the unmethylated CpG positions are represented by a TG or a CA, wherein CA is used when the strand is analyzed that is (or was before bisulfite treatment) reverse complementary to the strand containing the CpG position of interest.

To avoid contamination with the standard nucleic acid according to the invention from one experiment to another, it is advantageous to substitute at least one, most preferably all cytosines with uracil bases. This way, the standard nucleic acid can be degraded after an experiment using uracil N-glycosylase (UNG), also referred to as carry-over prevention, as known in the art.

In a preferred embodiment, the first sequence portion and/or the second sequence portion of the nucleic acid is flanked on either or on both sides by a primer sequence for binding of a primer that can initiate an amplification reaction, and/or a restriction site for cutting the nucleic acid to make it ligatable to another nucleic acid, e.g. inserting it into a plasmid using ligation. This way, the nucleic acid according to the invention can be constructed in a cassette-like structure. Then, each cassette preferably contains at least part of the sequence of the DNA molecule to be analyzed with at least one CpG position of interest. The location of recognition sites for a restriction enzyme on either or both sides flanking of the cassette allows the construction of a nucleic acid with several copies of the cassette that might differ in their sequence only at the CpG sites in that the CpG sites can represent either the methylated or the unmethylated status.

The primer sequence allows for the amplification of the first and the second sequence portion, in particular when the cassette-like structure is realized. Furthermore, the primer sequence can also be used for the binding of sequencing primers to confirm the sequence of the nucleic acid. It is furthermore possible that the restriction site and the primer sequence (partially) overlap.

In a preferred embodiment of the invention, the nucleic acid comprises a label for the detection of the nucleic acid. This is important, if the detection is performed using e.g. a microarray. For other detection methods, such as QM as described above, no label of the nucleic acid is necessary.

The label can be, e.g., a hapten such as biotin, digoxigenine; a fluorophore such as FAM, HEX, ROX, or Tamra. Furthermore, a quencher (e.g. BHQ1, BHQ1, Dabcyl), a protein or peptide label, a luminescent dye, an antigen, an antibody, a ligand, a ligand-binding molecule, a streptavidin molecule, an oligonucleotide, a radioactive label, a mass label, or a reagent.

In another preferred embodiment of the invention, the nucleic acid is an oligonucleotide, a probe, a primer, or, particularly preferred, a plasmid. Realization of the nucleic acid in the form of a plasmid allows for easy synthesis of the standard in large batches.

The problem underlying the present invention is furthermore solved by the use of a nucleic acid as described above as a standard for the quantitative bisulfite based analysis of the methylation status of a DNA molecule, in particular of a genomic DNA molecule. It is, however, also possible to use the nucleic acid on any DNA molecule for which the determination of the methylation status is of interest.

The problem underlying the present invention is also solved by a method for the quantitative analysis of the methylation status of a DNA molecule.

This method will typically be carried out by performing at least the following steps, preferably in the given order:

Firstly, the DNA molecule to be analyzed is treated so that the base pairing behavior of methylated cytosine bases and/or unmethylated cytosine bases of the DNA molecule are altered such that methylated cytosine bases become distinguishable from unmethylated cytosine bases. Preferably, treatment of the nucleic acid is performed with a chemical reagent or an enzyme containing solution. Most preferably, the treatment is performed with a bisulfite containing solution, so that unmethylated cytosine bases are converted into uracil bases, while methylated cytosine bases remain unchanged.

Secondly, the methylation status of the DNA molecule is analyzed using a) a first molecule for generating at least one detectable signal that allows to determine the methylation status of the DNA molecule, and b) a nucleic acid as described above as a standard, for generating at least one detectable control signal of the methylated status and for generating at least one detectable control signal of the unmethylated status.

Thirdly, the at least one signal generated by the first molecule, and the at least one control signal of the methylated status and the at least one control signal of the unmethylated status generated by the standard nucleic acid are detected.

Fourthly, the at least one signal is quantitated based on the at least one control signal of the methylated status and the at least one control signal of the unmethylated status.

In order to obtain reliable results with the method according to the invention, it is of importance that the first molecule and the standard nucleic acid are processed in parallel, that is under the same conditions.

In the first step of the method according to the invention, the DNA is chemically or enzymatically treated in such a way that all of the unmethylated cytosine bases are converted to uracil or another base which is dissimilar to cytosine in terms of base pairing behavior, while the 5-methylcytosine bases remain unchanged.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of performing said treatment are known in the art (e.g. PCT/EP 2004/011715).

It is preferred that the bisulfite treatment is conducted in the presence of denaturing solvents, such as, but not limited to, n-alkylenglycol, particularly diethylene glycol dimethyl ether (DME), or in the presence of dioxane or dioxane derivatives. In a preferred embodiment, the denaturing solvents are used in concentrations between 1 % and 35 % (v/v). It is also preferred that the bisulfite reaction is carried out in the presence of scavengers such as, but not limited to, chromane derivatives, e.g., 6-hydroxy-2,5,7,8-tetramethylchromane 2-carboxylic acid or trihydroxybenzoe acid and derivates thereof, e.g. Gallic acid (see: PCT/EP2004/011715). The bisulfite conversion is preferably carried out at a reaction temperature between 30 °C and 70 °C, whereby the temperature is increased to over 85 °C for short periods of times during the reaction (see: PCT/EP2004/011715). The bisulfite-treated DNA is preferably purified prior to the quantification. This may be conducted by any means known in the art, such as, but not limited to, ultrafiltration, preferably carried out by means of Microcon^{®} columns (manufactured by Millipore^{®}). The purification is carried out according to a modified manufacturer's protocol (see: PCT/EP2004/011715).

It is also possible to conduct the conversion enzymatically, e.g. by use of methylation-specific cytidine deaminases (German Patent DE 103 31 107; PCT/EP2004/007052).

The analysis of the methylation status of the DNA molecule using a first molecule for providing at least one detectable signal that allows to determine the methylation status of the DNA molecule can be performed using various methods known in the art. Various suite methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, and a number of PCR based methylation assays such as QM, Pyrosequencing and QAMA. Other suitable PCR based methylation assays - known as COBRA, MS-SNuPE, MSP, nested MSP, HeavyMethyl and MethyLight - are described in more detail now.

BISULFITE SEQUENCING. DNA methylation patterns and 5-methylcytosine distribution can be analyzed by sequencing analysis of a previously amplified fragment of the bisulfite treated genomic DNA, as described by Frommer et al. (Frommer et al. Proc. Natl. Acad. Sci. USA 89: 1827-1831, 1992).

COBRA. COBRA analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992) or as described by Olek et al. (Olek A, Oswald J, Walter J. (1996) Nucleic Acids Res. 24: 5064-6). PCR amplification of the bisulfite converted DNA is then performed using methylation unspecific primers followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from micro disected paraffin-embedded tissue samples.

Ms-SNuPE (Methylation-sensitive Single Nucleotide Primer Extension). The Ms-SNuPE technique is a quantitative method for assessing methylation differences at specific CpG sites based on bisulfite treatment of DNA, followed by single-nucleotide primer extension (Gonzalgo and Jones, Nucleic Acids Res. 25: 2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (e.g., micro disected pathology sections), and it avoids utilization of restriction enzymes for determining the methylation status at CpG sites.

MSP. Methylation-specific PCR allows for assessing the methylation status of virtually any group of CpG sites within a CpG island, independent of the use of methylation-sensitive restriction enzymes (Herman et al. Proc. Natl. Acad. Sci. USA 93: 9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA.

MSP primer pairs contain at least one primer that hybridizes to a bisulfite treated CpG dinucleotide. Therefore, the sequence of said primers comprises at least one CpG dinucleotide. MSP primers specific for non-methylated DNA contain a "T' at the 3' position of the C position in the CpG. Preferably, therefore, the base sequence of said primers is required to comprise a sequence having a length of at least 9 nucleotides which hybridizes to the bisulfite converted nucleic acid sequence, wherein the base sequence of said oligomers comprises at least one CpG dinucleotide. MSP requires only small quantities of DNA, is sensitive to 0.1 % methylated alleles of a given CpG island locus, and can be performed on DNA extracted from paraffin-embedded samples.

NESTED MSP (Belinsky and Palmisano in US application 20040038245). Considering the apparent conflict of requiring high specificity of the MSP primer to sufficiently differentiate between CG and TG positions but allowing for a mismatch in order to create a unique restriction site it is preferred to use an amended version of MSP, known as nested MSP, as described in WO 02/18649 and US patent application 20040038245 by Belinsky and Palmisano. This method to detect the presence of gene-specific promoter methylation, comprises the steps of expending the number of copies of the genetic region of interest by using a polymerase chain reaction to amplify a portion of said region where the promoter methylation resides, thereby generating an amplification product; and using an aliquot of the amplification product generated by the first polymerase chain reaction in a second, methylation-specific, polymerase chain reaction to detect the presence of methylation. In other words a non methylation specific PCR is performed prior to the methylation specific PCR.

HEAVYMETHYL. (WO 02/072880 ; Cottrell SE et al. Nucleic Acids Res. 2004 Jan 13;32(1):e10) A further preferred embodiment of the method comprises the use of blocker oligonucleotides. In the HeavyMethyl assay blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a `CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivatized at the 3' position with other than a free hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-termini thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker—a process that normally results in degradation of the hybridized blocker oligonucleotide. The use of peptide nucleic acid (PNA) oligomers as blocking oligonucleotides is preferred. Such PNA blocker oligomers are ideally suited, because they are neither decomposed nor extended by the polymerase.

Preferably, therefore, the base sequence of said blocking oligonucleotide is required to comprise a sequence having a length of at least 9 nucleotides which hybridizes to the chemically treated nucleic acid sequence, wherein the base sequence of said oligonucleotides comprises at least one CpG, TpG or CpA dinucleotide.

Preferably, real-time PCR assays are performed. Real-time PCR assays can be performed with methylation specific primers (MSP-real time) as methylation-specific PCR ("MSP"; as described above), or with non-methylation specific primers in presence of methylation specific blockers (HM real-time) ("HEAVYMETHYL", as described above). Real-time PCR may be performed with any suitable detectably labeled probes.

Both methods, MSP and HM, can be combined with a detection method known as MethyLight (a fluorescence-based real-time PCR technique) (Eads et al., Cancer Res. 59:2302-2306, 1999), which generally increases the specificity of the signal generated in such an assay. Whenever the real-time probe used is methylation specific in itself, the technology shall be referred to as MethyLight, a widely used method.

Another assay makes use of the methylation specific probe, the so called "QM" (quantitative methylation) assay. A methylation unspecific, therefore unbiased real-time PCR amplification is performed which is accompanied by the use of two methylation specific probes (MethyLight) one for the methylated and a second for the unmethylated amplificate. That way, two signals are generated which can be used to a) determine the ratio of methylated (CG) to unmethylated (TG) nucleic acids, and at the same time b) the absolute amount of methylated nucleic acids can be determined, when calibrating the assay with a known amount of control DNA.

MethyLight. The MethyLight assay is a high-throughput quantitative methylation assay that utilizes fluorescence-based real-time PCR (TaqMan^{®}) technology that requires no further manipulations after the PCR step (Eads et al., Cancer Res. 59: 2302-2306, 1999). Briefly, the MethyLight process begins with a mixed sample of genomic DNA that is converted in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not cover known methylation sites (a fluorescence-based version of the "MSP" technique), or with oligonucleotides covering potential methylation sites.

The MethyLight process can by used with a TaqMan^{®} probe in the amplification process. For example, double-stranded genomic DNA is treated with sodium bisulfite and subjected to one of two sets of PCR reactions using TaqMan^{®} probes; e.g., with either biased primers and TaqMan^{®} probe, or unbiased primers and TaqMan^{®} probe. The TaqMan^{®} probe is dual-labeled with fluorescent "reporter" and "quencher" molecules, and is designed to be specific for a relatively high GC content region so that it melts out at about 10 °C higher temperature in the PCR cycle than the forward or reverse primers. This allows the TaqMan^{®} probe to remain fully hybridized during the PCR annealing/extension step. As the Taq polymerase enzymatically synthesizes a new strand during PCR, it will eventually reach the annealed TaqMan^{®} probe. The Taq polymerase 5' to 3' endonuclease activity will then displace the TaqMan^{®} probe by digesting it to release the fluorescent reporter molecule for quantitative detection of its now unquenched signal using a real-time fluorescent detection system.

Variations on the TaqMan^{®} detection methodology that are also suitable for use with the described invention include the use of dual-probe technology (LightCycler^{®} or fluorescent amplification primers (Sunrise™ technology). Both these techniques may be adapted in a manner suitable for use with bisulfite treated DNA.

The fragments obtained by means of the amplification can carry a directly or indirectly detectable label. Preferred are labels in the form of fluorescence labels, radionuclides, or detachable molecule fragments having a typical mass, which can be detected in a mass spectrometer. Where said labels are mass labels, it is preferred that the labeled amplificates have a single positive or negative net charge, allowing for better detectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, (1988) Anal Chem 60: 2299-2301). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1: 147-157, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallization. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut and Beck, Nucleic Acids Res. 23: 1367-73, 1995). The coupling of a charge-tag to this modified DNA results in an increase in MALDI-TOF sensitivity to the same level as that found for peptides.

The amplificates may also be further detected and/or analyzed by means of oligonucleotides constituting all or part of an "array" or "DNA chip" (i.e., an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the prior art in oligomer array manufacturing can be gathered from Nature Genetics Supplement, Volume 21, January 1999, and from the literature cited therein. Fluorescence labeled probes are often used for the scanning of immobilized DNA arrays. The simple attachment of Cy3 and Cy5 dyes to the 5'-OH of the specific probe is particularly suitable for fluorescence labels. The detection of the fluorescence of the hybridized probes may be carried out, for example, via a confocal microscope. Cy3 and Cy5 dyes, besides many others, are commercially available.

The problem underlying the present invention is furthermore solved by a kit for the quantitative analysis of the methylation status of a DNA molecule.

Such a kit comprises:
a) a chemical reagent or an enzyme containing solution for treating the DNA molecule so that the base pairing behavior of methylated cytosine bases and/or unmethylated cytosine bases of the DNA molecule are altered such that methylated cytosine bases become distinguishable from unmethylated cytosine bases,
b) a first molecule for generating at least one detectable signal of the methylation status of the DNA molecule, and
c) a nucleic acid as described above as a standard, for providing at least one detectable control signal of the methylated status and for providing at least one detectable control signal of the unmethylated status.

Preferably, the chemical reagent in the kit for treating the DNA molecule is bisulfite.

The test kit may further comprise one or more of the additional components, such as:
- a denaturing reagent and/or solution, for example: dioxane or diethylene glycol dimethylether (DME) or any substance, which is suitable as described in WO 05/038051,
- a scavenger molecule, for example 6-hydroxy-2,5,7,8-tetramethylchromane 2-carboxylic acid or other scavengers as described in WO 01/98528 or WO 05/038051,
- at least one additional primer, which is suitable for the amplification of one or more DNA amplificates. The primer or primers can be modified, for example with a quencher and/or a label for detection as well known by a person skilled in the art like the dye FAM or the quencher BHQ black hole or dabcyl,
- a probe, which can be any probe, which can be used to specifically record the amplification of one or more amplificates for example in a real-time-assay, amongst others the probe or probes can be modified, for example with a quencher and/or a label for detection as well known by a person skilled in the art like the dye FAM or the quencher BHQ black hole or dabcyl,
- a blocker, which are nucleic acids and can be used to block the binding of a specific primer or the replication by DNA polymerase, amongst others the blocker or blockers can be modified, for example with a quencher and/or a label for detection as well known by a person skilled in the art like the dye FAM or the quencher BHQ black hole or dabcyl,
- a reaction buffer, which are suitable for a bisulfite treatment and/or a PCR reaction,
- a nucleotide, which can be dATP, dCTP, dTTG, dUTP and dGTP or any derivative of these nucleotides,
- MgCl₂ and/or any other magnesium salt, which can be used to carry out a DNA polymerase replication,
- a DNA polymerase, for example Taq polymerase or any other polymerase with or without proof-reading acitivity,
- a dye or a quencher, which can be used for the detection of amplificates, for example an intercalating dye like SYBR Green or a dye for linkage to a primer or probe or blocker like the dye FAM or the quencher BHQ black hole or dabcyl, and/or
- any reagent, solution, device and/or instruction which is useful for realization of a method according to the invention.

The methods and test kit disclosed here are preferably used for the diagnosis and/or prognosis of adverse events for patients or individuals, whereby diagnosis means diagnose of an adverse event, a predisposition for an adverse event and/or a progression of an adverse event.

These adverse events belong to at least one of the following categories: undesired drug interactions; cancer diseases; CNS malfunctions, damage or disease; symptoms of aggression or behavioral disturbances; clinical, psychological and social consequences of brain damage; psychotic disturbances and personality disorders; dementia and/or associated syndromes; cardiovascular disease, malfunction or damage; malfunction, damage or disease of the gastrointestinal tract; malfunction, damage or disease of the respiratory system; lesion, inflammation, infection, immunity and/or convalescence; malfunction, damage or disease of the body as an abnormality in the development process; malfunction, damage or disease of the skin, of the muscles, of the connective tissue or of the bones; endocrine and metabolic malfunction, damage or disease; headaches or sexual malfunction.

The methods and test kits also serve for distinguishing cell types and tissues or for investigating cell differentiation. They also serve for analyzing the response of a patient to a drug treatment.

The invention furthermore relates to a bacterial cell that contains a nucleic acid as described above, i.e. a nucleic acid according to the invention, preferably in the form of a plasmid.

### Description of the drawings

### Figure 1:

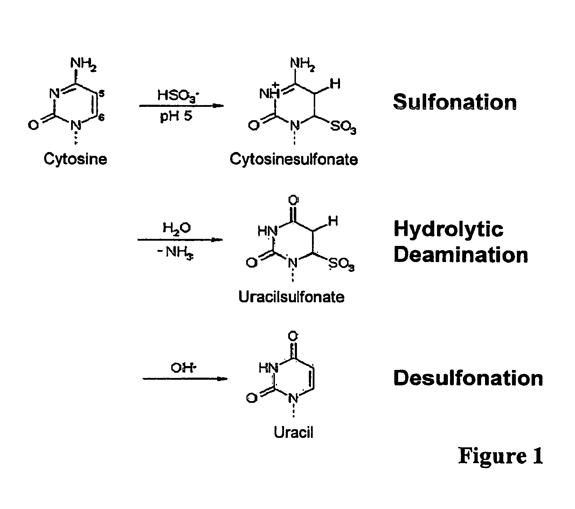

Figure 1 describes the complete conversion of unmethylated cytosine to uracil, also referred to as bisulfite conversion, which is known in the art. In the first step of this reaction, unmethylated cytosine bases are sulfonated at position C6 of the ring at a pH around 5 through reaction with hydrogensulfite.

The second step of the conversion is the deamination that takes place rather spontaneously in an aqueous solution. Thereby, cytosine sulfonate reacts to uracil sulfonate. The third step of the conversion is the desulfonation step, which takes place in alkaline conditions, resulting in uracil.

### Figure 2:

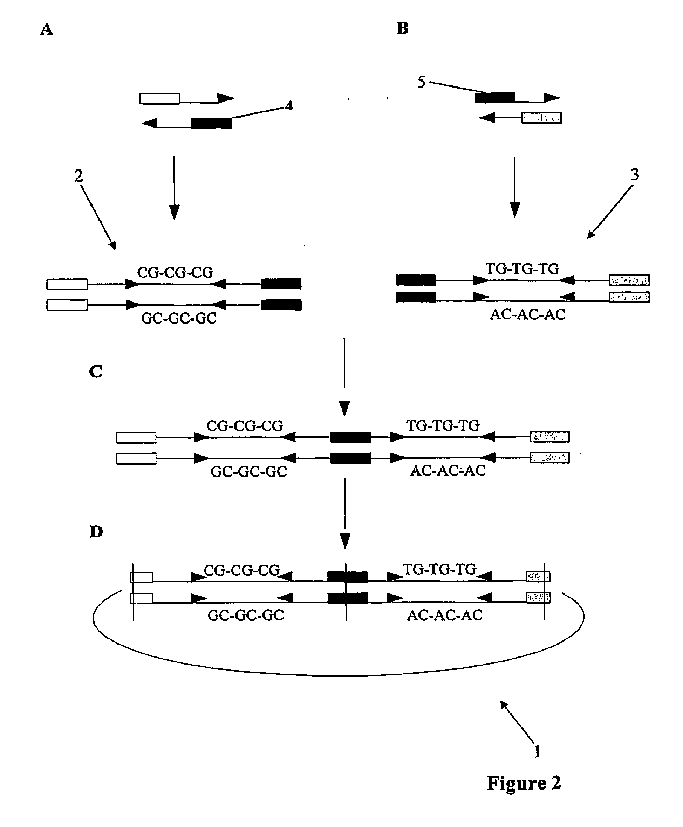

Figure 2 shows the synthesis of a nucleic acid 1 according to the invention in the form of a plasmid for providing a standard for the quantitative bisulfite based analysis of the methylation status of a DNA molecule. In the example shown, the nucleic acid 1 is constructed in a cassette-like structure, with two cassettes 2, 3 that each comprise the same sequence, namely the sequence of a DNA molecule containing at least one CpG position that is to be analyzed regarding its methylation status.

Part A of figure 2 shows the preparation of a first of theses cassettes 2 containing a first sequence portion, in which all of the three CpG positions are CG sequences, representing the methylated status. Part B of figure 2 shows the preparation of a second cassette 3 containing a second sequence portion, in which all of the three CpG positions are TG sequences (or CA sequences on the counter strand), representing the methylated status.

In the example shown, the first and the second sequence portion are amplified by a polymerase chain reaction (PCR). This PCR is performed on methylated (panel A) or unmethylated (panel B) template DNA using primers that contain a restriction site within them. In this example, one of the primers 4 used for amplifying the first sequence portion and one of the primers 5 used for amplifying the second sequence portion contain the same restriction site (represented by dark boxes in figure 2). This allows both cassettes 2, 3 to be ligated to each other following a restriction enzyme digest of the PCR products with a suitable restriction enzyme.

The resulting ligation product depicted in panel C of figure 2 contains a first cassette with the first sequence portion 2, in which all three of the CpG positions represent the methylated status, and a second cassette with the second sequence portion 3, in which all three of the CpG positions represent the unmethylated status. Therefore, the fixed ratio of the methylated over the unmethylated signal generated from the nucleic acid according to the invention will be 50 %. As will be apparent for a skilled person, using the strategy described, standard nucleic acids with different ratios can easily be synthesized.

It is noted that it might lead to recombination, if a nucleic acid 1 in the form of a plasmid is propagated in bacteria that contains more than one cassettes. Recombination might be avoided by introducing additional sequence portions between the cassettes. Also, certain strain of bacteria are available that are more tolerant for plasmids containing concatamer sequences. Such bacterial strains are known to a person skilled in the art.

Instead of using a PCR to generate the first and the second cassette 2, 3 in the necessary amount for cloning, it is also possible to synthesize oligonucleotide pairs with the appropriate sequences. The synthesized oligonucleotides (strand and counter strand) will then be hybridized to each other under conditions know to a person skilled in the art to form a double stranded DNA molecule that can then be used for the steps described below.

The ligation product shown in panel C of figure 2 can be ligated into a plasmid after both the plasmid and said ligation product have been digested with an appropriate restriction enzyme that cuts within the primer sequences on the ends of the ligation product and within e.g. a multiple cloning site of a plasmid vector. The resulting ligation product can be propagated in bacteria and isolated in practically any necessary amount. Prior to use, the sequence should be confirmed by sequencing.

Before the nucleic acid 1 is used as a standard, it is preferably cut. Depending on the detection method used, labeling of the nucleic acid 1 with a label might be necessary.

The length of the first and the second sequence portion can vary between 20 nucleotides to 2000 nucleotides, depending on the part of the DNA molecule that is analyzed for its methylation status.

### Figure 3:

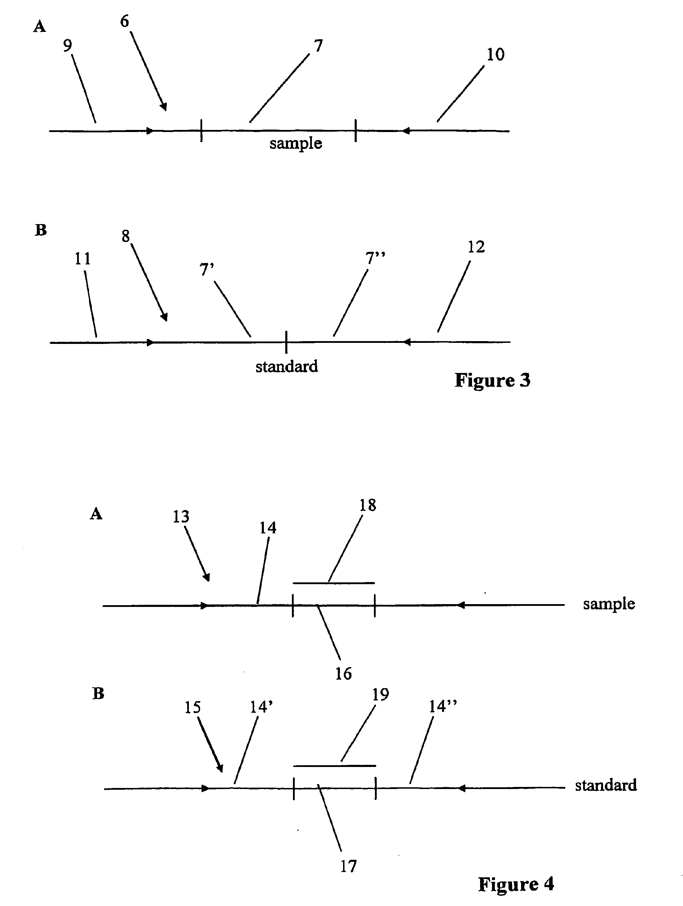

Figure 3A shows a target DNA molecule 6 ("sample") that is to be analyzed regarding its methylation status within a given sequence portion 7, containing at least one potentially methylated CpG position. The target DNA molecule 6 has been treated with bisulfite, converting all unmethylated cytosine bases into uracil bases.

Panel B of figure 3 depicts a nucleic acid 8 for providing a standard in the quantitative bisulfite based analysis of the methylation status of the target DNA molecule 6. The nucleic acid 8 comprises the same sequence as the sample twice, namely once in a first sequence portion 7' in a completely methylated status, and in a second sequence portion 7" in a completely unmethylated status.

For PCR amplification, the sample molecule 6 and the standard molecule 8 contain different sequence portions used as binding sites for oligonucleotide primers. The samples molecule 6 is flanked by a first primer sequence 9 and a second primer sequence 10, whereas the standard molecule 8 is flanked by a third primer sequence 11 and a fourth primer sequence 12. In a PCR reaction using four different primers binding to the first, second, third or fourth primer sequence 9, 10, 11, 12, the sample 6 and the standard molecule 8 are being amplified in a competing fashion. To enable signal detection by chip, the primers binding to the first and the third primer sequence are labeled with distinguishable dyes, such as Cy3 and Cy5. Then, normalization of the signal stemming from the sample can be normalized using the signal stemming from the standard, e.g. by calculating the Cy3/Cy5 signal ratio.

### Figure 4:

Figure 4A shows a target DNA molecule 13 ("sample") that is to be analyzed regarding its methylation status within a sequence portion 14, that contains at least one potentially methylated CpG position. The target DNA molecule 13 has been treated with bisulfite, so that all unmethylated cytosine bases have been converted into uracil bases.

Panel B of figure 4 depicts a nucleic acid 15 for providing a standard in the quantitative bisulfite based analysis of the methylation status of the target DNA molecule 13 (panel A of figure 4). The nucleic acid 15 comprises the same sequence portion as the sample, namely once in a completely methylated status (first sequence portion 14'), and in a completely unmethylated status (second sequence portion 14").

The sample molecule 13 and the standard molecule 15 are identical with respect to their sequence portions used as binding sites for oligonucleotide primers (shown by arrows). The differ, however, in a sequence portion 16, 17 that is used for hybridization to a probe 18, 19.

A first probe 18 can hybridize to a portion 16 of the sample molecule 13, whereas a second probe 19 can hybridize to a portion 17 of the standard molecule 15. Therefore, the addition of the standard molecule 15 as a template in a PCR amplification reaction of the sample molecule 13 will provide an internal control for the amplification reaction.

### Example

### Quality control of an oligonucleotide array

In this example, a quality control of an oligonucleotide array (Affymetrix chip) was performed using a standard nucleic acid according to the present invention. For this purpose, a portion of the PITX2 gene was chosen that contains three CpG sites.

The sequence of said standard nucleic acid, which was obtained through synthesis, was as follows:
Bio-gagtcgggagtcggagtcgggagagceaaeagt**TG**ggagt**TG**gagt**TG**ggagag**TG**aa (SEQ ID NO 1);
Bio: 5' biotinylation

The nucleic acid comprises a first sequence portion (underlined) and a second sequence portion (doubly underlined), both of which comprise the same part of the chosen PITX2 gene sequence with three CpG positions (bold). In the first sequence portion, all of the four CpG positions represent the methylated status (CG), and in the second sequence portion, all of the four CpG positions represent the unmethylated status (TG) after bisulfite conversion. Therefore, the standard nucleic acid comprises the first sequence portion and the second sequence portion in a fixed ratio, namely 1:1, mimicking a 50 % methylation mixture.

The standard nucleic acid shown above is used to calibrate an Affymetrix oligonucleotide array (EpiChip1d520483). For this purpose, signal intensity from hybridization of the standard nucleic acid to the following three oligonucleotides (1 to 3) located on said chip were measured. CG dinucleotides in oligonucleotides 1 and 2 and CA dinucleotides in oligonucleotide 3 are shown in bold.
1 T**CG**CTCTCC**CG**ACTC**CGA**CTCCC (SEQ ID NO 2)
2 TTT**CG**CTCTCC**CG**ACTC**CG**AC (SEQ ID NO 3)
3 TCCTTT**CA**CTCTCC**CA**ACTC**CA**ACT (SEQ ID NO 4)

The calibration of the oligonucleotide array was performed as follows:
The EpiChip1d520483 array is prehybridized for 10 min with 100 µl 2xSSPE, 0.05 % Triton, and 1x Denhardt's buffer. 100 µl calibrator oligonucleotide mixture (4 pmol/l in 2xSSPE; 0.05 % Triton; 1x Denhardt's containing Affymetrix oligo B2 (30 pmol/1)) is denatured for 10 min at 99°C, chilled on ice and subsequently 80 µl are used for hybridization at 56 °C in a hybridization oven for 1 h. Then, the hybridization buffer is discarded and the array is filled with washing buffer (100 µl 6xSSPE; 0.005 % Triton). The array is transferred into a Affymetrix fluidic station FS 450 and staining buffers are connected to the fluidics station: streptavidine buffer (streptavidine 0.1 mg/ml; 6xSSPE; 0.01 % Triton; 1x Denhardt's); Anti-Streptavidine-Antibody buffer (biotinylated anti-streptavidine-antibody 0.05 mg/ml; 6xSSPE; 0.01 % Triton; 1x Denhardt's); SAPE buffer (Streptavidin Phycoerytrin 0.1 mg/ml; 6xSSPE; 0.01 % Triton; 1x Denhardt's). Then a standard washing program is used (Micro_1v1_450) to wash the microarray. After washing the array is scanned using a gene chip 3000 DX scanner and the signal intensities of the three probes 1 to 3 complementary to the portion of the PITX2 sequence that is part of the standard nucleic acid are detected.

Log signal intensities of oligonucleotide 1 / log signal intensity of oligonucleotide 3, and log signal intensities of oligonucleotide 2 / log signal intensity of oligonucleotide 3 were calculated. If the values obtained are between 0.9 and 1.1, the array can be used.

## Claims

1. A nucleic acid for providing a standard in the quantitative bisulfite based analysis of the methylation status of a DNA molecule with a given sequence, wherein the given sequence comprises at least one CpG position that is to be analyzed for its methylation status,
wherein the nucleic acid comprises:
a first and a second sequence portion, both of which comprise at least part of the given sequence with at least one CpG position, wherein
- in the first sequence portion, all of the CpG positions represent the methylated status, and
- in the second sequence portion, all of the CpG positions represent the unmethylated status.

2. The nucleic acid according to claim 1, wherein the first and the second sequence portion comprise the same part of the given sequence.

3. The nucleic acid according to claim 1 or 2, comprising more than one first sequence portion and/or more than one second sequence portion.

4. The nucleic acid according to any of claims 1 to 3, comprising the first sequence portion and the second sequence portion in a fixed ratio, preferably in a fixed ratio of the first sequence portion to the second sequence portion of 1:1, 2:1, 3:1, 1:2, or 1:3.

5. The nucleic acid according to any of claims 1 to 4, wherein the methylated CpG positions are represented by a CG and/or the unmethylated CpG positions are represented by a TG or a CA.

6. The nucleic acid according to any of claims 1 to 5, wherein the first sequence portion and/or the second sequence portion is flanked by
- a primer sequence for binding of a primer that can initiate an amplification reaction, and/or
- a restriction site for cutting the nucleic acid to make it ligatable to another nucleic acid.

7. The nucleic acid according to claim 6, wherein the restriction site is part of the primer sequence.

8. The nucleic acid according to any of claims 1 to 7, comprising a label for the detection of the nucleic acid.

9. The nucleic acid according to any of claims 1 to 8, wherein the nucleic acid is an oligonucleotide, a probe, a primer, or a plasmid.

10. Use of a nucleic acid according to any of claims 1 to 9 as a standard for the quantitative bisulfite based analysis of the methylation status of a DNA molecule, in particular of genomic DNA.

11. A method for the quantitative analysis of the methylation status of a DNA molecule, comprising the following steps:
a. treating the DNA molecule so that the base pairing behavior of methylated cytosine bases and/or unmethylated cytosine bases of the DNA molecule are altered such that methylated cytosine bases become distinguishable from unmethylated cytosine bases, and
b. analyzing the methylation status of the DNA molecule using:
- a first molecule for providing at least one detectable signal that allows to determine the methylation status of the DNA molecule, and
- a nucleic acid according to any of claims 1 to 9 as a standard, for providing at least one detectable control signal of the methylated status and for providing at least one detectable control signal of the unmethylated status, and
c. detecting the at least one signal provided by the first molecule, as well as the at least one control signal of the methylated status and the at least one control signal of the unmethylated status provided by the standard nucleic acid, and
d. quantitating the at least one signal based on the at least one control signal of the methylated status and the at least one control signal of the unmethylated status.

12. The method according to claim 11, wherein treatment of the nucleic acid is performed with a chemical reagent or an enzyme containing solution, preferably with a bisulfite containing solution, such that unmethylated cytosine bases are converted into uracil bases, while methylated cytosine bases remain unchanged.

13. The method according to any of claims 11 or 12, wherein the first molecule and the standard nucleic acid are processed in parallel.

14. A kit for the quantitative analysis of the methylation status of a DNA molecule comprising:
a. a chemical reagent, preferably bisulfite, or an enzyme containing solution for treating the DNA molecule so that the base pairing behavior of methylated cytosine bases and/or unmethylated cytosine bases of the DNA molecule are altered such that methylated cytosine bases become distinguishable from unmethylated cytosine bases,
b. a first molecule for generating at least one detectable signal of the methylation status of the DNA molecule, and
c. a nucleic acid according to claims 1 to 9 as a standard, for providing at least one detectable control signal of the methylated status and for providing at least one detectable control signal of the unmethylated status.

15. A bacterial cell containing a nucleic acid according to claims 1 to 9.

## Patentansprüche

1. Nukleinsäure zur Bereitstellung eines Standards in der quantitativen bisulfid-basierten Analyse des Methylierungsstatus eines DNA Moleküls mit einer gegebenen Sequenz, wobei die gegebene Sequenz mindestens eine CpG Position, welche auf ihren Methylierungsstatus hin analysiert werde soll, umfasst, wobei die Nukleinsäure umfasst:
eine erste und eine zweite Sequenzportion, welche beide mindestens einen Teil der gegebenen Sequenz mit mindestens einer CpG Position umfassen, wobei
- in der ersten Sequenzportion alle CpG Positionen den methylierten Status repräsentieren, und
- in der zweiten Sequenzportion alle CpG Positionen den unmethylierten Status repräsentieren.

2. Nukleinsäure nach Anspruch 1, wobei die erste und die zweite Sequenzportion den gleichen Teil der gegebenen Sequenz umfassen.

3. Nukleinsäure nach Anspruch 1 oder 2, umfassend mehr als eine erste Sequenzportion und/oder mehr als eine zweite Sequenzportion.

4. Nukleinsäure nach einem der Ansprüche 1 bis 3, umfassend die erste Sequenzportion und die zweite Sequenzportion in einem festgelegten Verhältnis, vorzugsweise in einem festgelegten Verhältnis der ersten Sequenzportion zur zweiten Sequenzportion von 1:1, 2:1, 3:1, 1:2, oder 1:3.

5. Nukleinsäure nach einem der Ansprüche 1 bis 4, wobei die methylierten CpG Positionen durch ein CG repräsentiert sind und/oder die unmethylierten CpG Positionen durch ein TG oder ein CA repräsentiert sind.

6. Nukleinsäure nach einem der Ansprüche 1 bis 5, wobei die erste Sequenzportion und/oder die zweite Sequenzportion von
- einer Primer Sequenz zur Bindung eines Primers, welcher eine Amplifikations-Reaktion initiieren kann, und/oder
- einer Restriktionsstelle zum Schneiden der Nukleinsäure um diese mit einer anderen Nukleinsäure legierbar zu machen,
flankiert ist.

7. Nukleinsäure nach Anspruch 6, wobei die Restriktionsstelle Teil der Primersequenz ist.

8. Nukleinsäure nach einem der Ansprüche 1 bis 7, umfassend ein Kennzeichen zur Detektion der Nukleinsäure.

9. Nukleinsäure nach einem der Ansprüche 1 bis 8, wobei die Nukleinsäure ein Oligonucleotid, eine Sonde, ein Primer oder ein Plasmid ist.

10. Verwendung einer Nukleinsäure nach einem der Ansprüche 1 bis 9 als ein Standard in der quantitativen, bisulfid-basierten Analyse des Methylierungsstatus eines DNA Moleküls, insbesondere von genomischer DNA.

11. Verfahren zur quantitativen Analyse des Methylierungsstatus eines DNA Moleküls, umfassend die folgenden Schritte:
(a) Behandlung des DNA Moleküls, so dass das Basenpaarungsverhalten der methylierten Cytosin Basen und/oder unmethylierten Cytosin Basen des DNA Moleküls so verändert ist, dass methylierte Cytosin Basen von unmethylierten Cytosin Basen unterscheidbar werden,
(b) Analyse des Methylierungsstatus des DNA Moleküls unter Verwendung:
- eines ersten Moleküls zur Bereitstellung mindestens eines detektierbaren Signals, welches die Bestimmung des Methylierungsstatus des DNA Moleküls ermöglicht, und
- einer Nukleinsäure nach einem der Ansprüche 1 bis 9 als ein Standard zur Bereitstellung mindestens eines detektierbaren Kontrollsignals des methylierten Status und zur Bereitstellung mindestens eines detektierbaren Kontrollsignals des unmethylierten Status, und
(c) Detektierung mindestens eines Signals bereitgestellt von dem ersten Molekül sowie des mindestens einem Kontrollsignals des methylierten Status und des mindestens einem Kontrollsignals des unmethylierten Status bereitgestellt von der Standardnukleinsäure, und
(d) Quantifizierung des mindestens einem Signals auf der Grundlage des mindestens einem Kontrollsignals des methylierten Status und des mindestens einem Kontrollsignals des unmethylierten Status.

12. Verfahren nach Anspruch 11, wobei die Behandlung der Nukleinsäure mit einer Lösung, die ein chemisches Reagenz oder ein Enzym enthält, vorzugsweise mit einer bisulfidhaltigen Lösung, durchgeführt wird, so dass unmethylierte Cytosin Basen in Uracil Basen umgewandelt werden, während methylierte Cytosin Basen unverändert bleiben.

13. Verfahren nach einem der Ansprüche 11 oder 12, wobei das erste Molekül und die Standardnukleinsäure parallel verarbeitet werden.

14. Kit zur quantitativen Analyse des Methylierungsstatus eines DNA Moleküls, umfassend
(a) ein chemisches Reagenz, vorzugsweise Bisulfid, oder eine ein Enzym enthaltenden Lösung zur Behandlung des DNA Moleküls, so dass das Basenpaarungsverhalten der methylierten Cytosin Basen und/oder unmethylierten Cytosin Basen des DNA Moleküls so verändert ist, dass methylierte Cytosin Basen von unmethylierten Cytosin Basen unterscheidbar werden,
(b) ein erstes Molekül zur Erzeugung mindestens eines detektierbaren Signal des Methylierungsstatus des DNA Moleküls, und
(c) eine Nukleinsäure nach den Ansprüchen 1 bis 9 als ein Standard zur Bereitstellung mindestens eines detektierbaren Kontrollsignals des methylierten Status und zur Bereitstellung mindestens eines detektierbaren Kontrollsignals des unmethylierten Status.

15. Bakterielle Zelle beinhaltend eine Nukleinsäure nach den Ansprüchen 1 bis 9.

## Revendications

1. Acide nucléique pour procurer un étalon dans l'analyse quantitative sur la base du bisulfite de l'état de méthylation d'une molécule d'ADN ayant une séquence donnée, où la séquence donnée comprend au moins une position CpG qui doit être analysée quant à son état de méthylation,
dans lequel l'acide nucléique comprend:
une première et une deuxième portion de séquence, comprenant toutes deux au moins une partie de la séquence donnée avec au moins une position CpG, tandis que
- dans la première portion de séquence, la totalité des positions CpG représentent l'état méthylé, et
- dans la deuxième portion de séquence, la totalité des positions CpG représentent l'état non-méthylé.

2. Acide nucléique selon la revendication 1, dans lequel la première et la deuxième portion de séquence comprennent la même partie de la séquence donnée.

3. Acide nucléique selon la revendication 1 ou 2, comprenant plus d'une première portion de séquence et/ou plus d'une deuxième portion de séquence.

4. Acide nucléique selon l'une quelconque des revendications 1 à 3, comprenant la première portion de séquence et la deuxième portion de séquence dans un rapport fixé, de préférence dans un rapport fixé de la première portion de séquence à la deuxième portion de séquence de 1:1, 2:1, 3:1,1:2 ou 1:3.

5. Acide nucléique selon l'une quelconque des revendications 1 à 4, dans lequel les positions CpG méthylées sont représentées par un CG et/ou les positions CpG non-méthylées sont représentées par un TG ou un CA.

6. Acide nucléique selon l'une quelconque des revendications 1 à 5, dans lequel la première portion de séquence et/ou la deuxième portion de séquence est encadrée par
- une séquence d'amorce pour la liaison d'une amorce pouvant initier une réaction d'amplification, et/ou
- un site de restriction pour la coupure de l'acide nucléique pour le rendre apte à être ligaturé à un autre acide nucléique.

7. Acide nucléique selon la revendication 6, dans lequel le site de restriction fait partie de la séquence d'amorce.

8. Acide nucléique selon l'une quelconque des revendications 1 à 7, comprenant un marqueur pour la détection de l'acide nucléique.

9. Acide nucléique selon l'une quelconque des revendications 1 à 8, dans lequel l'acide nucléique est un oligonucléotide, une sonde, une amorce, ou un plasmide.

10. Utilisation d'un acide nucléique selon l'une quelconque des revendications 1 à 9 à titre d'étalon pour l'analyse quantitative sur la base du bisulfite de l'état de méthylation d'une molécule d'ADN, en particulier d'ADN génomique.

11. Procédé pour l'analyse quantitative de l'état de méthylation d'une molécule d'ADN, comprenant les étapes suivantes:
a. traitement de la molécule d'ADN de manière à altérer le comportement d'appariement de bases des bases cytosine méthylées et/ou des bases cytosine non-méthylées de la molécule d'ADN, de telle sorte que les bases de cytosine méthylées puissent être distinguées des bases de cytosine non-méthylées, et
b. analyse de l'état de méthylation de la molécule d'ADN, avec utilisation de:
- une première molécule pour procurer au moins un signal détectable permettant la détermination de l'état de méthylation de la molécule d'ADN, et
- un acide nucléique selon l'une quelconque des revendications 1 à 9 à titre d'étalon, pour procurer au moins un signal témoin détectable de l'état méthylé et pour procurer au moins un signal détectable de l'état non-méthylé, et
c. détection du au moins un signal procuré par la première molécule, ainsi que du au moins un signal témoin de l'état méthylé et du au moins un signal témoin de l'état non-méthylé procurés par l'acide nucléique étalon, et
d. quantification du au moins un signal sur la base du au moins un signal témoin de l'état méthylé et du au moins un signal témoin de l'état non-méthylé.

12. Procédé selon la revendication 11, dans lequel le traitement de l'acide nucléique est effectué avec une solution contenant un réactif chimique ou une enzyme, de préférence avec une solution contenant du bisulfite, de telle manière que les bases cytosine non-méthylées sont converties en bases uracile, tandis que les bases cytosine méthylées restent inchangées.

13. Procédé selon l'une quelconque des revendications 11 ou 12, dans lequel la première molécule et l'acide nucléique étalon sont traités en parallèle.

14. Kit pour l'analyse quantitative de l'état de méthylation d'une molécule d'ADN, comprenant:
a. une solution contenant un réactif chimique, de préférence du bisulfite, ou une enzyme, pour le traitement de la molécule d'ADN, de telle sorte que le comportement d'appariement de bases des bases cytosine méthylée et/ou des bases cytosine non-méthylée de la molécule d'ADN sont altérées de telle manière que les bases cytosine méthylée deviennent aptes à être distinguées des bases cytosine non-méthylée,
b. une première molécule pour la génération d'au moins un signal détectable de l'état de méthylation de la molécule d'ADN, et
c. un acide nucléique selon les revendications 1 à 9 à titre d'étalon, pour procurer au moins un signal témoin détectable de l'état méthylé et pour procurer au moins un signal témoin détectable de l'état non-méthylé.

15. Cellule bactérienne contenant un acide nucléique selon les revendications 1 à 9.


### SEQUENCE LISTING

<110> EPIGENOMICS AG
<120> A molecule for providing a standard for the quantitative analysis of the methylation status of a nucleic acid
<130> E60092PCT
<150> EP 06123628.7
   <151> 2006-11-07
<150> EP 06122484.6 <151> 2006-10-18
<160> 4
<170> PatentIn version 3.3
<210> 1
   <211> 58
   <212> DNA
   <213> Homo sapiens
<400> 1
   gagtcgggag tcggagtcgg gagagcgaag agttgggagt tggagttggg agagtgaa 58
<210> 2
   <211> 23
   <212> DNA
   <213> Homo sapiens
<400> 2
   tcgctctccc gactccgact ccc 23
<210> 3
   <211> 21
   <212> DNA
   <213> Homo sapiens
<400> 3
   tttcgctctc ccgactccga c 21
<210> 4
   <211> 25
   <212> DNA
   <213> Homo sapiens
<400> 4
   tcctttcact ctcccaactc caact 25